# EUROPEAN PATENT SPECIFICATION

(11) **EP 2 762 097 B1**
(45) Date of publication and mention of the grant of the patent: **22.03.2017**
(21) Application number: 13153466.1
(22) Date of filing: 31.01.2013
(51) Int. Cl.: A61B 17/74, A61B 17/86

(54) **Modular lag screw**
Modulare Verzögerungsschraube
Vis tire-fond modulaire

(43) Date of publication of application: 06.08.2014
(73) Proprietor: Stryker European Holdings I, LLC, Kalamazoo, MI 49002 (US)
(72) Inventor: Probe, Robert A., Temple, TX Texas 76502 (US); Paulsen, Martje, 24103 Kiel (DE); Simon, Bernd, 24107 Kiel (DE)
(74) Representative: Schmitz, Alexander

(56) References cited:
- EP-A1- 2 343 020
- JP-A- 2009 207 710
- US-A1- 2007 270 847
- US-A1- 2008 177 334
- US-A1- 2008 281 326
- US-A1- 2009 326 534
- US-A1- 2011 066 152

## Description

### FIELD OF THE DISCLOSURE

The invention relates to an implant. In particular, the invention relates to a bone implant like a lag screw to be received in a transverse through bore in a bone nail or hip plate.

### BACKGROUND INFORMATION

An implant and particularly a bone implant include a portion or section or end which is adapted to be firstly introduced into a body during an implantation. In the specification, such a portion or section or end is referred to as leading portion or leading section or leading end. Consequently, an opposite portion or section or end of the implant is adapted to be introduced last, wherein this portion or section or end may additionally be configured for an engagement of a tool for inserting the implant into the body. Below, such a portion or section or end is referred to as trailing portion or trailing section or trailing end.

A bone implant may be a pin or a nail or screw. A bone nail may be an intramedullary nail, for example a femur nail, a humerus nail or a tibia nail. A bone screw may be a screw for fixing fragments of a bone fracture or may be a locking screw for locking a bone nail in the bone.

US 2007/0270847 describes a compression hip screw, wherein a locking plug has a flat face for abutment against the lag screw.

US 2008/0177334 describes a compression screw with a proximal screw part engaging into a distal screw part so as to pull the distal screw part in a longitudinal direction, and a spine fixation system including a screw for engagement of a bone and a locking nut for rotationally stable securing a cross member or an extension member to the screw. US 2008/0281326 describes a lag screw comprising an outer sleeve and a screw which can be accommodated within the sleeve and can be moved relative to the sleeve. US2011/0066152 describes a lag screw, wherein a proximal part of the screw is linked to a distal part of the screw by means of at least one member by means of which an angle between the proximal and distal parts of the screw can be adjusted.

EP 2 343 020 describes a bone screw being an assembly of a tubular shaft and a head, the head being configured to engage with a driver to advance the bone screw into a bone by rotating the driver, wherein the head is connected in such a way to the tubular shaft that it is locked against disconnection when the driver is rotated.

US2009/0326534 describes a modular lag screw according to the preamble of claim 1.

However, due to the anatomical variation of bones it may happen that the trailing end of a bone implant extends out of a bone after implant placement. The trailing end of the implant may act as an interface with an implantation tool and may thus be provided with an appropriate structure like for example slots for controlling the forces applied during implant insertion and removal. It may occur that patients complain about pain after surgery in this area, especially when the implant is extending out of the bone. This pain may be caused by sharp edges at the trailing end of the implant. Such edges may cause irritation and/or injury to the surrounding soft tissue.

A migration of a hip screw is reported in the case report "Hip screw lateral migration with no cut-out or non-union implication: a case report" of Nikolaos Lasanianos et al. (Case Journal 2009, 2:6419). A similar report can be found in "Medial pelvic migration of the lag screw in a short gamma nail after hip fracture fixation: a case report and review of the literature" of Xinning Li et al. (Journal of Orthopaedic Surgery and Research 2010, 5:62).

### SUMMARY OF THE INVENTION

One aspect of the invention may be defined as providing an implant causing less irritation of surrounding tissue when being implanted.

This is achieved by the bone screw according to the independent claim. Further embodiments are described in the dependent claims.

In general, an elongated implant like a bone screw according to an embodiment is a modular screw which has a distal part having a leading end and a trailing end, designated herein as a first leading end and a first trailing end. The first trailing end includes a tool engagement portion designated herein as first tool engagement portion. The modular implant further has a proximal part also having a leading end and a trailing end. The ends of the proximal part are designated herein as a second leading end and a second trailing end, respectively, wherein the second leading end includes an engagement portion for an engagement with the tool engagement portion of the first trailing end of the distal implant part, therefore designated as distal part engagement portion, and wherein the second trailing end also includes a tool engagement portion wherein this one is designated as second tool engagement portion. The implant further comprises an assembly element, such as a screw, for firmly coupling the distal part and the proximal part when the first tool engagement portion of the distal part engages the distal part engagement portion of the proximal part.

In other words, the implant comprises two parts which can be assembled so as to form a single implant, wherein the distal part is adapted to be introduced into a body first and the proximal part is adapted to follow the distal part during an implantation and thus to be introduced into the body last. It is noted that the distal part may be used as an implant without the proximal part.

Such a combination of parts as an implant allows the surgeon to shorten the implant once protrusion of the implant out of for example a bone has occurred due to bone fragment compression. Such a shortening may be accomplished in a short procedure leaving at least the distal part of the implant in place which may be crucial as the probability of removal, patient outcome and stability are influenced by the implant. Accordingly, an implant is provided which can be assembled pre-operatively with a distal part and a proximal part, either at the time of manufacture or in the operating room, and can be disassembled by a smaller surgery merely removing the proximal part.

According to an embodiment, the first tool engagement portion of the distal part comprises at least one slot and the distal part engagement portion of the proximal part comprises at least one protrusion for an engagement with the at least one slot so that particularly rotational forces can be transmitted from the proximal part to the distal part. It will be understood that the protrusion may have any suitable shape, for example a shape of a circular or rectangular pin, of a prong, spike or tooth.

According to an embodiment, the first tool engagement portion of the distal part is crown-shaped. It is noted that the distal part engagement portion may have any shape which is suitable to transmit forces, in particular rotational forces from the proximal part to the distal part.

According to an embodiment, the distal part engagement portion of the proximal part is formed so as to provide a form-fit with the tool engagement portion of the distal part. It can be seen as an advantage of a form-fit connection that no gaps or free edges may exist between the distal and proximal parts.

The trailing end of the proximal part is provided with a tool engagement portion corresponding to the tool engagement portion of the trailing end of the distal part. Accordingly, the distal part engagement portion at the leading end of the proximal part may be formed for an engagement with the tool engagement portion at the trailing end of the distal part, and the trailing end of the proximal part may comprise a second tool engagement portion which corresponds to the first tool engagement portion.

Such a proximal part can be understood as an extension of the distal part as a tool which fits to the first tool engagement portion of the distal part may also fit into the second tool engagement portion of the proximal part. It will be understood that it may be possible to further extend the length of the implant by an additional proximal part when coupling the leading end of the additional proximal part to the trailing end of another proximal part. Of course proximal parts of different length can be provided.

According to an embodiment, the implant is a bone implant, in particular a bone screw and the proximal part comprises an end surface which corresponds to a shape of an outer bone surface at an intended implantation site and is smooth so that the end surface of the implant can be flush with the bone surface surrounding the end surface when the implant is inserted into the bone. For example, the end surface may be curved in one direction to fit to the shape of an outer surface of a more or less circular long bone when implanted substantially perpendicular to the bone axis, like for example a locking screw of an intramedullary nail, or the end surface may be shaped as a saddle to match the outer surface at a greater trochanter of a femur. Alternatively, the end surface may be inclined with respect to a longitudinal axis of the implant when the implant, for example a lag screw, is introduced into a bone with an angle relative to the outer surface of the bone.

It will be understood that "smooth" refers to a shape without any edges, in particular without any sharp edge. That is, the end surface may be formed without any discontinuities. The edge between the end surface and an circumferential outer surface of the implant may be provided with a chamfer or may be rounded so as to be also smooth, i.e. so as to not form any edge at which irritation of soft tissue may occur when the tissue is in contact with a trailing section of the implant including the end surface.

According to another embodiment, the distal part comprises an axial bore with an inner thread at the first trailing end, and the proximal part comprises an axial through bore with an enlarged section, and the assembly screw comprises a screw head being adapted to be received in the enlarged section in the proximal part and an outer screw thread being adapted to engage the inner thread in the distal part so as to firmly couple the distal part and the proximal part.

According to an embodiment, the distal part and the proximal part are configured to move over a guide wire when the implant is assembled, i.e. when the first tool engagement portion of the distal part engages the distal part engagement portion of the proximal part. Accordingly, the implant may further comprise a cannulation or through bore extending in a longitudinal direction of the shaft, for accommodating a guide wire. It has to be noted that a person skilled in the art will gather from the above and the following description that, unless otherwise notified, in addition to any combination of features belonging to one embodiment, also any combination of features relating to another embodiment is considered to be disclosed with this application.

These and other objects, features and advantages of the exemplary embodiments of the present invention will become apparent upon reading the following detailed description of exemplary embodiments, when taken in conjunction with the appended claims.

### BRIEF DESCRIPTION OF THE DRAWINGS

The invention will now be detailed by way of exemplary embodiments with reference to the attached drawings.
Figure 1A is a sectional view, figure 1B is a side view and figure 1C is a top view of a distal part of a bone screw.
Figure 2A is an isometric view and figure 2B is a sectional view of a proximal part according to a first embodiment.
Figure 3A is a sectional view and figure 3B is a side view of a proximal part according to an example.
Figure 4A is a sectional view and figure 4B is a side view of an assembly element according to a first embodiment.
Figure 5A is a sectional view and figure 5B is a side view of an assembly element according to a second embodiment.
Figures 6A-C illustrate a migration of a lag screw.
Figure 7 shows a modular lag screw with a smooth end surface.
Figures 8A and 8B show an embodiment of a modular bone screw being assembled and disassembled, respectively.

It is noted that the illustrations in the drawings are only schematically and not to scale. Throughout the drawings, the same reference numerals and characters, unless otherwise stated, are used to denote like features, elements, components, or portions of the illustrated embodiments. Moreover, while the present invention will now be described in detail with reference to the figures, it is done so in connection with the illustrative embodiments and is not limited by the particular embodiments illustrated in the figures, as defined by the appended claims.

### DETAILED DESCRIPTION OF EXEMPLARY EMBODIMENTS

Embodiments of an implant are shown in the figures and described below, comprising a bone screw as a distal part, two different proximal parts as bone screw extensions as well as two different assembly screws for coupling at least one of the proximal parts with the distal part.

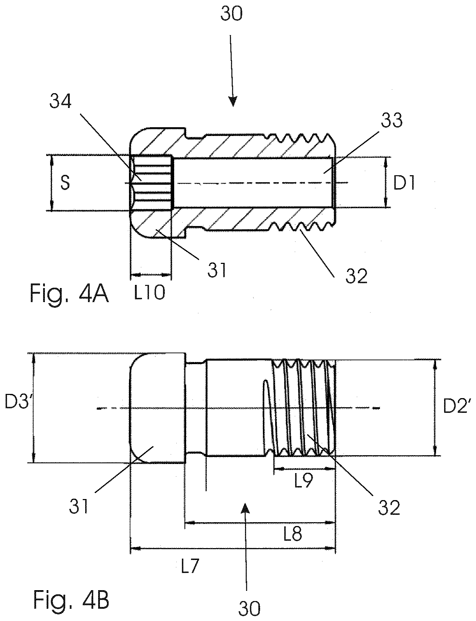

In general, the distal part 10 as well as the assembly element 30 comprises an axial through bore 13 having a diameter D1, for accommodating a guide wire. At the trailing section of the distal part 10, a threaded bore with an outer diameter D2 is provided, for receiving outer threads of an assembly element 30 having a corresponding diameter D2' (figure 4B). The proximal part 20 also includes a bore section with a diameter D2 allowing an assembly element 30 to extend through that bore section of the proximal part 20. Furthermore, the proximal part 20 includes an enlarged bore section having a diameter D3 for accommodating a head 31 of the assembly element 30 having an outer diameter D3' which is slightly smaller than the diameter D3 in the enlarged bore section of the proximal part to easily fit into that bore section. Referred to as D4 is an outer diameter of an inner thread in the proximal part 20 for accommodating a threaded screw head 31 with a diameter D4' which should also be slightly smaller to easily fit into that inner thread. Finally, an outer diameter D5 denotes an overall outer diameter of the implant, that is, of the distal part as well as of any of the proximal parts.

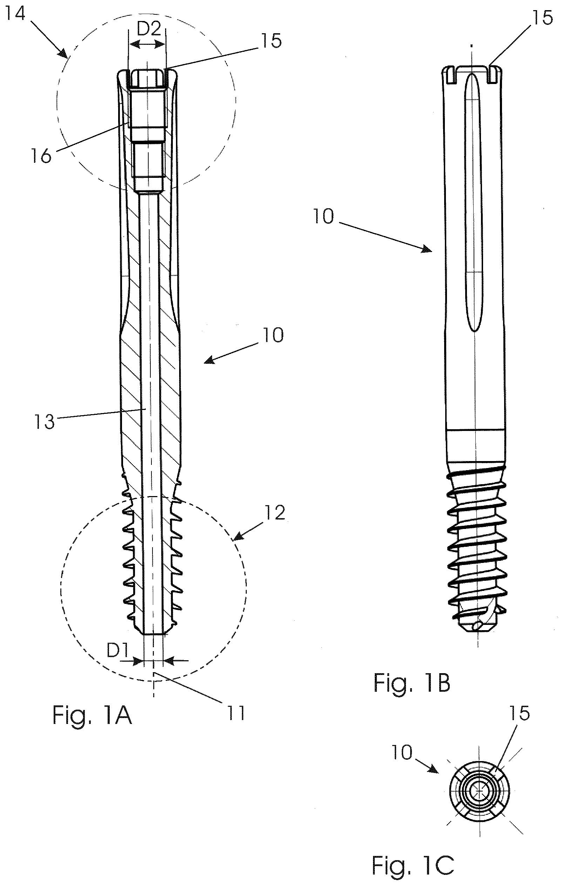

As shown in figures 1A, 1B and 1C, a distal part 10 may comprise a longitudinal axis 11, a leading end section 12, an axial through bore 13, a trailing end section 14, a tool engagement portion 15 as well as a threaded bore 16. At the leading end section 12, an outer thread is provided so that the implant can be screwed in into a bone. The axial through bore 13 is a central through bore for accommodating a guide wire. In the trailing end section 14, the axial bore has an enlarged portion in which an inner thread is provided. The threaded bore 16 is provided for receiving an assembly element in case a proximal part 20 is to be coupled to the trailing end of the distal part 10. The tool engagement portion 15 can be described as crown-shaped having four slots which extend in a longitudinal direction of the distal part 10 and which are evenly, i.e. every 90 degrees, arranged on the circumference at the trailing end section 14 of the distal part 10.

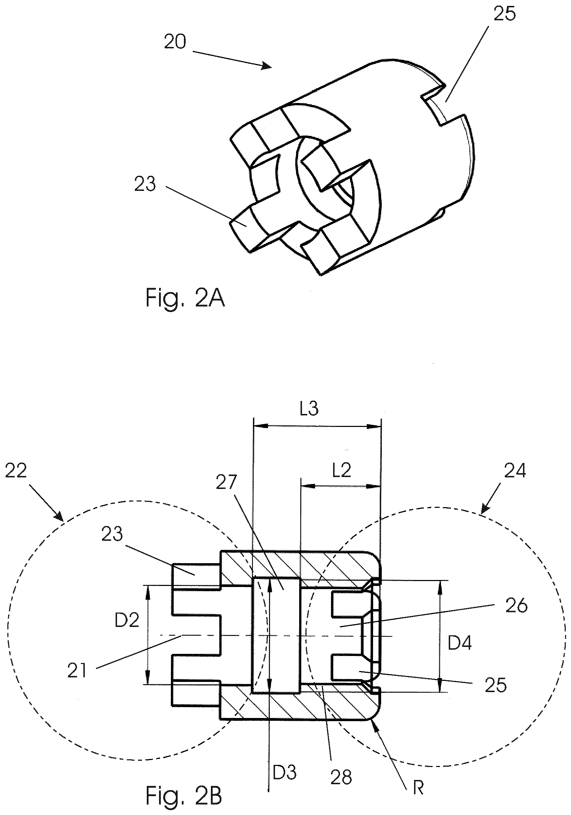

Figures 2A and 2B show a first embodiment of a proximal part 20. The proximal part 20 comprises a leading end section 22 as well as a trailing end section 24. The leading end section 22 comprises four protrusions 23 which are configured to fit into the slots of the tool engagement portion 15 at the trailing end section 14 of the distal part 10. At the opposite end, i.e. at the trailing end section 24, slots 25 are provided forming a tool engagement portion which corresponds to the tool engagement portion 15 of the distal part 10. Furthermore, the proximal part 20 comprises a through bore 26 extending along a longitudinal axis 21, wherein the through bore 26 includes an enlarged section 27. By the enlarged section 27, a shoulder is formed on which a head of an assembly element 30 may abut so that the proximal part 20 may be firmly coupled to the distal part 10 when an assembly element 30 is inserted into the proximal part with the head abutting at the shoulder formed by the enlarged section 27 and with a threaded portion extending into the distal part and in particular into the threaded bore 16 of the distal part 10.

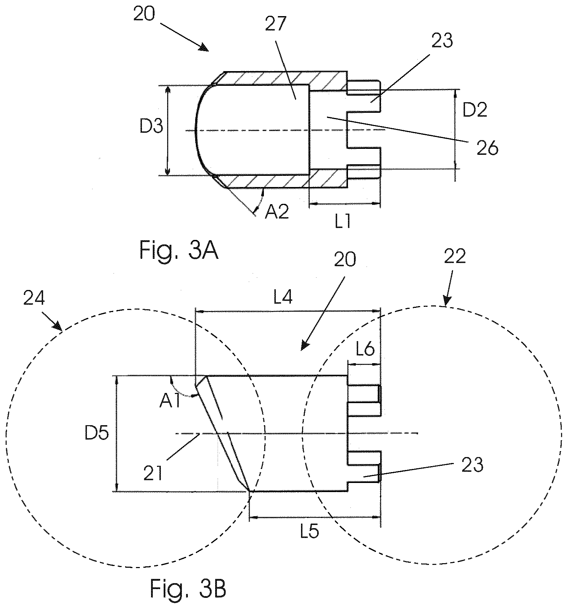

Figures 3A and 3B show an example of a proximal part 20. The proximal part 20 comprises a leading end section 22 and a trailing end section 24 wherein the leading end section 22 is formed comparable with the leading end section 22 of the first embodiment of a proximal part as shown in figures 2A and 2B. At the trailing end section 24, an inclined end surface is formed, with an inclination angle A1. An implant with a proximal part 22 having an inclined end surface and a distal part 10 can be introduced into a bone with an angle A1 relative to the outer bone surface so that the end surface of the proximal part being arranged so as to form a flush surface with the surrounding outer bone surface when the implant is implanted. Furthermore, the outer edge of the end surface may have a chamfer with an angle A2 relative to the outer circumferential surface of the proximal part.

Figures 4A and 4B show a first embodiment of an assembly element 30 which is in this example an assembly screw 30. The assembly screw 30 comprises an outer thread 32 as well as a head 31. Furthermore the assembly screw 30 is cannulated, i.e. comprises an axial through bore 33. In the head 31 of the assembly screw 30, an inner tool engagement portion is provided, for example for a screwdriver. It will be understood, that the width across flats S is preferably greater than the diameter D1 of the through bore 33. The outer diameter D2' should be configured to fit with a loose fit to the diameter D2 of the inner thread of the bore 16 in the distal part 10. The outer diameter D3' of the head 31 of the assembly screw 30 should be configured to fit with a loose fit to the diameter D3 of the enlarged section in the proximal part 20.

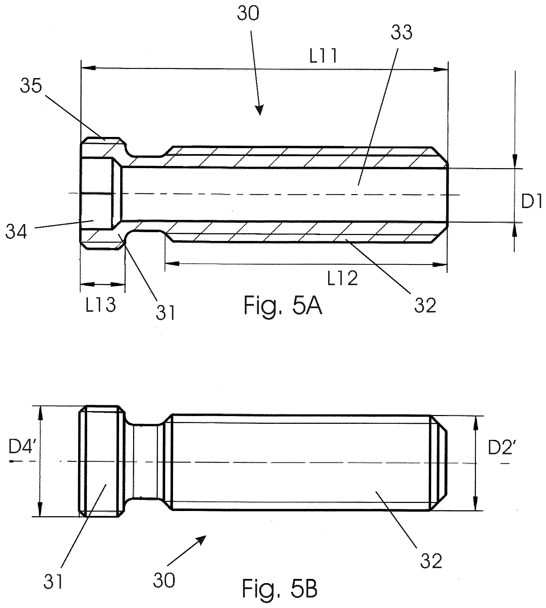

Figures 5A and 5B show a second embodiment of an assembly screw 30 with an outer thread 32 and a head 31. As the assembly screw 30 shown in figures 4A and 4B, the assembly screw of figures 5A and 5B comprises a through bore 33 as well as an inner tool engagement portion 34. Additionally, the second embodiment of the assembly screw 30 of figures 5A and 5B comprises an additional outer thread 35 on the outer surface of the head 31. The additional outer thread 35 may be used as a blocking thread when the assembly screw 30 is inserted into the distal and proximal parts. The outer thread 35 may engage with an inner threaded portion 28 in the proximal part 20 as shown in figure 2B, so as to secure the assembly screw 30 within the distal and proximal parts.

In the following, examples of dimensions are provided for the dimensions denoted in the figures with the respective reference signs.

The inner diameter D1 may be in the range between 3 mm and 4 mm, for example 3.4 mm.

The inner diameter D2, D2' may be in the range between 5 mm and 7.5 mm, for example a norm diameter of a metric thread M5, M6 or M7.

The inner diameter D3, D3' may be in the range between 6 mm and 9 mm, for example 8.1 mm.

The inner diameter D4, D4' may be in the range between 6 mm and 8 mm, for example a norm diameter of a metric thread M6 or M7.

The outer diameter D5 may be in the range between 9 mm and 11 mm, for example 10.5 mm.

The length L1 may be in the range between 5 mm and 9 mm, for example 6.5 mm.

The length L2 may be in the range between 4 mm and 8 mm, for example 5 mm.

The length L3 may be in the range between 6 mm and 11 mm, for example 8 mm.

The length L4 may be in the range between 15 mm and 20 mm, for example 17.5 mm.

The length L5 may be in the range between 10 mm and 14 mm, for example 12 mm.

The length L6 may be in the range between 2.5 mm and 3.5 mm, for example 3 mm.

The length L7 may be in the range between 10 mm and 30 mm, for example 15 mm or 23 mm.

The length L8 may be in the range between 8 mm and 14 mm, for example 11 mm.

The length L9 may be in the range between 3 mm and 20 mm, for example 4.5 mm or 17.7 mm.

The length L10 may be in the range between 2.5 mm and 3.5 mm, for example 3 mm.

The length L11 may be in the range between 2.5 mm and 5 mm, for example 2.8 mm or 4 mm.

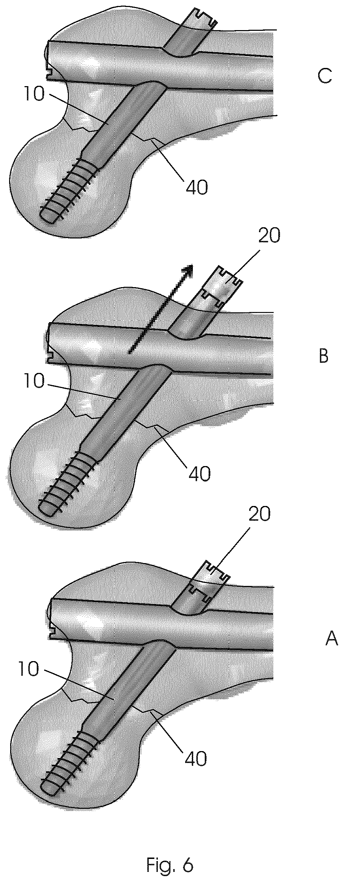

Figures 6A-C illustrate an application of a modular lag screw inserted through a bone nail and into a head of a femur. In figure 6A, a combination of a distal part 10 with an outer thread at its leading end portion and a proximal part 20 as a screw extension is inserted into a femur. Under load, it may occur that the bone at the fracture site 40 is compressed. As a result, the lag screw migrates in the direction of the arrow in figure 6B so that the trailing end of the modular lag screw protrudes out of the bone. Such a protrusion may cause irritation in the surrounding soft tissue. As depicted in figure 6C, the proximal part 20 of the modular lag screw may be removed. This may be done without removing the distal part 10 thus not effecting the stabilization of the femur neck at the fracture site 40.

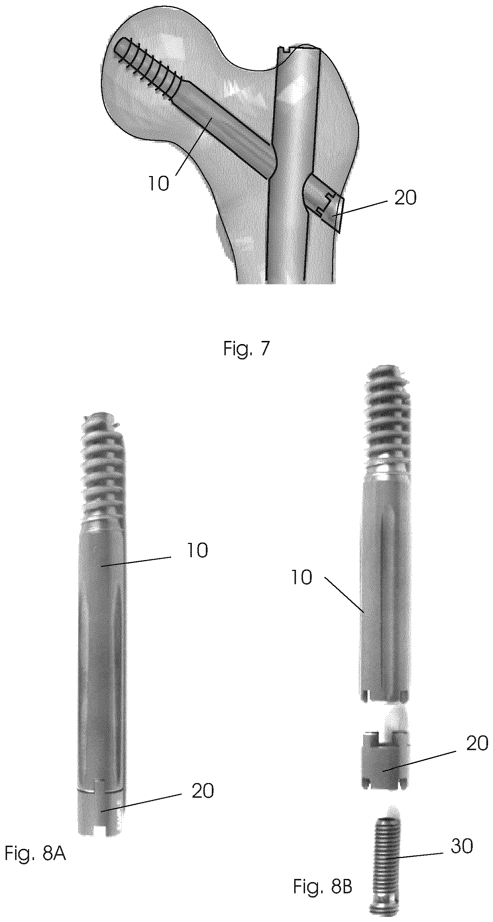

Figure 7 shows an embodiment of a modular lag screw with a proximal part 20 having a smooth trailing end surface. The smooth end surface may be configured so as to form substantially the contour of the outer bone surface surrounding the trailing end of the lag screw when implanted, here the outer surface of the shaft portion of the femur at the location at which the lag screw has been introduced into the femur.

Figure 8A shows an embodiment of a modular bone screw with a distal part 10 and a proximal part 20 in an assembled condition. Figure 8B shows an embodiment of a modular bone screw with a distal part 10 and a proximal part 20 in a disassembled condition. Figure 8B further shows assembly element 30 which is adapted to be introduced through the proximal part 20 and into the distal part 10 to firmly couple the proximal part and the distal part.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practising the claimed invention, according to the appended claims.

In the claims, the word "comprising" does not exclude other elements, and the indefinite article "a" or "an" does not exclude a plurality.

The mere fact that the certain measures are recited in mutually different dependent claims does not indicate that a combination of these measures cannot be used to advantage. Any reference signs in the claims should not be construed as limiting the scope.

### LIST OF REFERENCE SIGNS

- 10: distal part
- 11: longitudinal axis
- 12: first leading end section
- 13: axial bore
- 14: first trailing end section
- 15: first tool engagement portion
- 16: inner thread
- 20: proximal part
- 21: longitudinal axis
- 22: second leading end section
- 23: distal part engagement portion
- 24: second trailing end section
- 25: second tool engagement portion
- 26: axial bore
- 27: enlarged section
- 28: inner thread
- 30: assembly element
- 31: screw head
- 32: outer thread
- 33: axial bore
- 34: inner tool engagement portion
- 35: outer thread
- 40: bone fracture

## Claims

1. A modular lag screw comprising
a distal part (10) comprising a first leading end (12) and a first trailing end (14), wherein the first trailing end includes a first tool engagement portion (15), and
a proximal part (20) comprising a second leading end (22) and a second trailing end (24), wherein the second leading end includes a distal part engagement portion (23) configured for an engagement with the first tool engagement portion of the first trailing end of the distal part and for transmitting rotational forces from the proximal part to the distal part, and wherein the second trailing end includes a second tool engagement portion (25), and
an assembly element (30) for firmly coupling the distal part (10) and the proximal part (20) when the first tool engagement portion (15) of the distal part engages the distal part engagement portion (23) of the proximal part,
**characterized in that**
the first tool engagement portion (15) corresponds to the second tool engagement portion (25).

2. The screw of claim 1, wherein the first tool engagement portion (15) comprises at least one slot and the distal part engagement portion (23) comprises at least one protrusion for an engagement with the at least one slot.

3. The screw of claim 1 or 2, wherein the first tool engagement portion (15) is crown-shaped.

4. The screw of any one of claims 1 to 3, wherein the distal part engagement portion (23) of the proximal part is formed so as to provide a form fit with the tool engagement portion (15) of the distal part.

5. The screw of any one of claims 1 to 4, wherein the distal part engagement portion (23) at the second leading end (22) of the proximal part is formed for an engagement with the tool engagement portion (15) at the first trailing end (14) of the distal part, and wherein the second trailing end (24) of the proximal part comprises a second tool engagement portion (25) which corresponds to the first tool engagement portion (15).

6. The screw of any one of claims 1 to 4, wherein the proximal part (20) comprises an end surface at the second trailing end section (24) which corresponds to a shape of an outer bone surface at an intended implantation site so that the end surface of the bone screw is flush with the bone surface surrounding the end surface when the bone screw is inserted into the bone.

7. The screw of claim 6, wherein the end surface at the second trailing end section (24) is inclined with respect to a longitudinal axis (11, 21) of the bone screw.

8. The screw of any one of claims 1 to 7, wherein the distal part (10) comprises an axial bore (13) with an inner thread (16) at the first trailing end, and the proximal part (20) comprises an axial through bore (26) with an enlarged section (27), wherein the assembly screw (30) comprises a screw head (31) being adapted to be received in the enlarged section (27) in the proximal part and an outer screw thread (33) being adapted to engage the inner thread (16) in the distal part so as to firmly couple the distal part and the proximal part.

9. The screw of any one of claims 1 to 8, wherein the distal part (10) and the proximal part (20) are configured to move over a guide wire when the first tool engagement portion (15) of the distal part engages the distal part engagement portion (23) of the proximal part.

## Patentansprüche

1. Eine modulare Schraube aufweisend
ein distales Teil (10), welches ein erstes vorderes Ende (12) und ein erstes hinteres Ende (14) aufweist, wobei das erste hintere Ende einen ersten Werkzeugeingriffsabschnitt (15) umfasst, und
ein proximales Teil (20), welches ein zweites vorderes Ende (22) und ein zweites hinteres Ende (24) aufweist, wobei das zweite vordere Ende einen Eingriffsabschnitt (23) für das distale Teil umfasst, der für einen Eingriff in den ersten Werkzeugeingriffsabschnitt des ersten hinteren Endes des distalen Teils und für die Übertragung von Drehkräften von dem proximalen Teil auf das distale Teil ausgelegt ist, und wobei das zweite hintere Ende einen zweiten Werkzeugeingriffsabschnitt (25) umfasst, und
ein Montageelement (30) zur festen Verbindung des distalen Teils (10) und des proximalen Teils (20), wenn der erste Werkzeugeingriffsabschnitt (15) des distalen Teils in den Eingriffsabschnitt (23) für das distale Teil des proximalen Teils eingreift,
**dadurch gekennzeichnet, dass**
der erste Werkzeugeingriffsabschnitt (15) dem zweiten Werkzeugeingriffsabschnitt (25) entspricht.

2. Die Schraube gemäß Anspruch 1, wobei der erste Werkzeugeingriffsabschnitt (15) mindestens eine Aussparung aufweist und der Eingriffsabschnitt (23) für das distale Teil mindestens einen Vorsprung für einen Eingriff in die mindestens eine Aussparung aufweist.

3. Die Schraube gemäß Anspruch 1 oder 2, wobei der erste Werkzeugeingriffsabschnitt (15) kronenförmig ist.

4. Die Schraube gemäß einem der Ansprüche 1 bis 3, wobei der Eingriffsabschnitt (23) des proximalen Teils geformt ist, um einen Formschluss mit dem Werkzeugeingriffsabschnitt (15) des distalen Teils zu bilden.

5. Die Schraube gemäß einem der Ansprüche 1 bis 4, wobei der Eingriffsabschnitt (23) für das distale Teil an dem zweiten vorderen Ende (22) des proximalen Teils für einen Eingriff in den Werkzeugeingriffsabschnitt (15) an dem ersten hinteren Ende (14) des distalen Teils geformt ist und wobei das zweite hintere Ende (24) des proximalen Teils einen zweiten Werkzeugeingriffsabschnitt (25) aufweist, der dem ersten Werkzeugeingriffsabschnitt (15) entspricht.

6. Die Schraube gemäß einem der Ansprüche 1 bis 4, wobei das proximale Teil (20) eine Stirnfläche an dem zweiten hinteren Endabschnitt (24) aufweist, die einer Form einer äußeren Knochenoberfläche an einer vorgesehenen Implantationsstelle entspricht, sodass die Stirnseite der Knochenschraube mit der die Stirnseite umgebenden Knochenoberfläche bündig ist, wenn die Knochenschraube in den Knochen eingeführt ist.

7. Die Schraube gemäß Anspruch 6, wobei die Stirnfläche an dem zweiten hinteren Endabschnitt (24) gegenüber einer Längsachse (11, 21) der Knochenschraube geneigt ist.

8. Die Schraube gemäß einem der Ansprüche 1 bis 7, wobei das distale Teil (10) eine axiale Bohrung (13) mit einem Innengewinde (16) am ersten hinteren Ende aufweist und das proximale Teil (20) eine axiale Durchgangsbohrung (26) mit einem vergrößerten Bereich (27) aufweist, wobei die Montageschraube (30) einen Schraubenkopf (31) aufweist, der dazu ausgeführt ist, in den vergrößerten Bereich (27) in dem proximalen Teil aufgenommen zu werden und ein Außengewinde (33), das dazu ausgeführt ist, in das Innengewinde (16) in dem distalen Teil einzugreifen, um das distale Teil und das proximale Teil fest zu verbinden.

9. Die Schraube gemäß einem der Ansprüche 1 bis 8, wobei das distale Teil (10) und das proximale Teil (20) derart ausgeführt sind, dass sie sich über einen Führungsdraht bewegen, wenn der erste Werkzeugeingriffsabschnitt (15) des distalen Teils in den Eingriffsabschnitt (23) für das distale Teil des proximalen Teils eingreift.

## Revendications

1. Vis tire-fond modulaire comportant :
une partie distale (10) comportant une première extrémité avant (12) et une première extrémité arrière (14), dans laquelle la première extrémité arrière comprend une première portion de prise d'outil (15), et
une partie proximale (20) comportant une seconde extrémité avant (22) et une seconde extrémité arrière (24), dans laquelle la seconde extrémité avant comprend une portion de prise de partie distale (23) configurée pour une prise avec la première portion de prise d'outil de la première extrémité arrière de la partie distale et pour transmettre des forces de rotation de la partie proximale à la partie distale, et dans laquelle la seconde extrémité arrière comprend une seconde portion de prise d'outil (25), et
un élément d'assemblage (30) pour coupler fermement la partie distale (10) et la partie proximale (20) lorsque la première portion de prise d'outil (15) de la partie distale vient en prise avec la portion de prise de partie distale (23) de la partie proximale,
**caractérisée en ce que**
la première portion de prise d'outil (15) correspond à la seconde portion de prise d'outil (25).

2. Vis selon la revendication 1, dans laquelle la première portion de prise d'outil (15) comporte au moins une fente et la portion de prise de partie distale (23) comporte au moins une saillie pour une mise en prise avec la au moins une fente.

3. Vis selon la revendication 1 ou 2, dans laquelle la première portion de prise d'outil (15) est en forme de couronne.

4. Vis selon l'une quelconque des revendications 1 à 3, dans laquelle la portion de prise de partie distale (23) de la partie proximale est formée de manière à assurer un emboîtement de formes avec la portion de prise d'outil (15) de la partie distale.

5. Vis selon l'une quelconque des revendications 1 à 4, dans laquelle la portion de prise de partie distale (23) sur la seconde extrémité avant (22) de la partie proximale est formée pour une mise en prise avec la portion de prise d'outil (15) sur la première extrémité arrière (14) de la partie distale, et dans laquelle la seconde extrémité arrière (24) de la partie proximale comporte une seconde portion de prise d'outil (25) qui correspond à la première portion de prise d'outil (15).

6. Vis selon l'une quelconque des revendications 1 à 4, dans laquelle la partie proximale (20) comporte une surface d'extrémité sur la seconde section d'extrémité arrière (24) qui correspond à une forme d'une surface osseuse extérieure sur un site d'implantation prévu de sorte que la surface d'extrémité de la vis à os affleure la surface osseuse entourant la surface d'extrémité lorsque la vis à os est insérée dans l'os.

7. Vis selon la revendication 6, dans laquelle la surface d'extrémité sur la seconde section d'extrémité arrière (24) est inclinée par rapport à un axe longitudinal (11, 21) de la vis à os.

8. Vis selon l'une quelconque des revendications 1 à 7, dans laquelle la partie distale (10) comporte un alésage axial (13) pourvu d'un filet intérieur (16) sur la première extrémité arrière, et la partie proximale (20) comporte un alésage traversant axial (26) ayant une section élargie (27), dans laquelle la vis d'assemblage (30) comporte une tête de vis (31) adaptée pour être reçue dans la section élargie (27) dans la partie proximale et un filet de vis extérieur (33) étant adapté pour venir en prise avec le filet intérieur (16) dans la partie distale de manière à fermement coupler la partie distale et la partie proximale.

9. Vis selon l'une quelconque des revendications 1 à 8, dans laquelle la partie distale (10) et la partie proximale (20) sont configurées pour se déplacer sur un fil-guide lorsque la première portion de prise d'outil (15) de la partie distale vient en prise avec la portion de prise de partie distale (23) de la partie proximale.
